# DEMANDE DE BREVET EUROPEEN

(11) **EP 2 317 306 A1**
(43) Date de publication de la demande: **04.05.2011**
(21) Numéro de dépôt: 10188772.7
(22) Date de dépôt: 25.10.2010
(51) Int. Cl.: G01N 27/414, G01N 33/543

(54) **Détecteur de matière biologique ou chimique et matrice de détecteurs correspondante**

(30) Priorité: 30.10.2009 FR 0957688
(71) Demandeur: STMicroelectronics (Crolles 2) SAS, 38920 Crolles (FR)
(72) Inventeur: Monfray, Stéphane, 38320, EYBENS (FR); Skotnicki, Thomas, 38920, CROLLES-MONTFORT (FR)
(74) Mandataire: de Beaumont, Michel

(57) **Abrégé**

Détecteur de matière biologique ou chimique, comprenant un transistor MOS dont la région de canal (30) de silicium monocristallin est insérée entre des grilles isolées supérieure (32) et inférieure (25) la grille supérieure étant isolée de la région de canal par un isolant de grille supérieure (34) et la grille inférieure étant isolée du canal par un isolant de grille inférieure, la grille isolée supérieure comprenant une couche de détection adaptée à générer une charge à l'interface de la grille isolée supérieure et de son isolant de grille, l'épaisseur de l'isolant de grille supérieure (34) étant inférieure à l'épaisseur de l'isolant de grille inférieure (23).

## Description

### Domaine de l'invention

La présente invention concerne le domaine des circuits intégrés. Elle concerne plus particulièrement le domaine de la détection de matière biologique ou chimique.

### Exposé de l'art antérieur

Pour détecter la présence de matière biologique ou chimique dans un environnement et quantifier sa concentration, on a proposé d'utiliser un détecteur constitué d'une puce semiconductrice revêtue d'une couche d'un matériau susceptible de se lier à ladite matière.

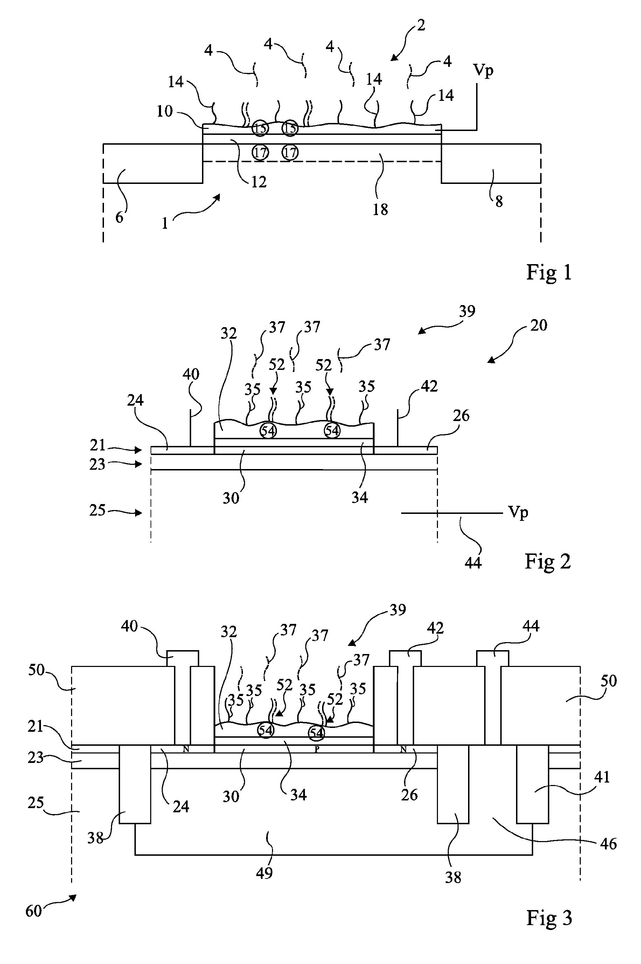

La figure 1 représente un tel détecteur 1 disposé dans un environnement aqueux 2. L'environnement aqueux 2 contient des molécules 4 dont on souhaite quantifier la concentration dans le milieu 2. Le détecteur 1 est un transistor MOS comprenant des régions de source 6 et de drain 8 ainsi qu'une couche de grille 10 et un isolant de grille 12. La grille isolée 10 est une couche de détection comprenant des liaisons pendantes 14.

Lorsqu'une molécule 4 s'apparie avec une liaison pendante 14, il apparaît une charge électrique 15 dans la couche de grille 10. L'apparition de cette charge électrique 15 génère l'apparition d'une charge 17 électriquement opposée dans la région de canal 18 du transistor 1, la grille 10 étant par ailleurs polarisée à une tension Vp proche de la tension de seuil du transistor. L'apparition de la charge 17 modifie la tension de grille du transistor 1 donc le courant source-drain. En quantifiant cette modification, on en déduit la concentration de molécules 4 dans le milieu 2.

Un tel détecteur 1 nécessite la présence d'une électrode externe permettant de polariser la grille 10 à la tension Vp. La reproductibilité ainsi que la fiabilité des mesures de concentration ne sont pas assurées. En outre, le niveau de sensibilité d'un tel détecteur n'est pas suffisant. Lorsqu'on a une quantité faible de matière à détecter, la différence de tension générée par l'apparition des charges 17 ne permet pas de modifier suffisamment la tension de grille du transistor.

### Résumé

Un objet d'un mode de réalisation de la présente invention est de prévoir un détecteur de matière biologique ou chimique évitant au moins certains des inconvénients des détecteurs antérieurs.

Un objet plus particulier d'un mode de réalisation de la présente invention est de prévoir un détecteur capable d'effectuer des mesures de concentration de matière biologique ou chimique fiables et répétitives.

Un autre objet d'un mode de réalisation de la présente invention est de prévoir un détecteur autonome et intégré à un substrat semi-conducteur.

Ainsi, un mode de réalisation de la présente invention prévoit un détecteur de matière biologique ou chimique, comprenant un transistor MOS dont la région de canal est insérée entre des grilles isolées supérieure et inférieure, la grille isolée supérieure comprenant une couche de détection adaptée à générer une charge à l'interface de la grille isolée supérieure et de son isolant de grille, l'épaisseur de l'isolant de grille supérieure étant inférieure à l'épaisseur de l'isolant de grille inférieure.

Selon un autre mode de réalisation de la présente invention, le rapport entre l'épaisseur de l'isolant de grille inférieure et l'épaisseur de l'isolant de grille supérieure est compris entre 2 et 20, de préférence entre 8 et 10.

Selon un autre mode de réalisation de la présente invention, l'épaisseur de l'isolant de grille supérieure est comprise entre 0,5 et 5 nm, de préférence entre 2 et 3 nm.

Selon un autre mode de réalisation de la présente invention, l'épaisseur de l'isolant de grille inférieure est comprise entre 5 et 50 nm, de préférence entre 15 et 25 nm.

Selon un autre mode de réalisation de la présente invention, l'épaisseur de la région de canal est définie de sorte que les grilles supérieure et inférieure soient couplées de manière capacitive.

Selon un autre mode de réalisation de la présente invention, l'épaisseur de la région de canal est comprise entre 1 et 40 nm, de préférence entre 2 et 20 nm.

Selon un autre mode de réalisation de la présente invention, la grille isolée inférieure est reliée électriquement à une électrode de contact disposée sur la face avant.

Selon un autre mode de réalisation de la présente invention, la grille inférieure est constituée d'un caisson fortement dopé relié à l'électrode de contact par un puits.

Un autre mode de réalisation de la présente invention prévoit une matrice de détecteurs comprenant une pluralité de détecteurs tels que décrits ci-dessus.

Selon un autre mode de réalisation de la présente invention, au moins deux détecteurs comprennent une couche de détection distincte.

### Brève description des dessins

Ces objets, caractéristiques et avantages, ainsi que d'autres seront exposés en détail dans la description suivante de modes de réalisation particuliers faite à titre non-limitatif en relation avec les figures jointes parmi lesquelles :
la figure 1, précédemment décrite, est une vue en coupe schématique d'un détecteur ;
la figure 2 est une vue en coupe schématique de principe d'un détecteur selon un mode de réalisation ; et
la figure 3 est une vue en coupe schématique d'un détecteur selon un autre mode de réalisation de la présente invention.

### Description détaillée

Par souci de clarté, de mêmes éléments ont été désignés par de mêmes références aux différentes figures et, de plus, comme cela est habituel dans la représentation des circuits intégrés, les diverses figures ne sont pas tracées à l'échelle.

La figure 2 est une vue schématique de principe représentant un détecteur 20 fabriqué dans un substrat semiconducteur de type SOI (Silicon On Isolator) comprenant une couche de silicium monocristallin mince 21 sur une couche isolante 23, par exemple de l'oxyde de silicium, reposant sur une tranche de silicium 25. Un transistor MOS comportant des régions de source 24 et de drain 26 de part et d'autre d'une région de canal 30 est réalisé dans une portion de la couche 21. Un isolant de grille 34 est disposé au-dessus de la région de canal 30. Une grille isolée supérieure 32 est disposée sur l'isolant 34. A titre d'exemple, l'isolant de grille 34 peut être en oxyde de silicium. La tranche 25 constitue une grille isolée inférieure du transistor, la couche isolante 23 constituant un isolant de grille inférieure.

La grille isolée supérieure 32 est constituée d'une couche de détection comprenant des liaisons pendantes 35. Les liaisons 35 sont adaptées à s'apparier avec des molécules 37 présentes dans un milieu 39 dans lequel le détecteur 20 peut être placé.

Les régions de source et de drain 24 et 26 sont respectivement reliées à des électrodes de contact 40 et 42. De la même manière, la grille isolée inférieure est reliée électriquement à une électrode de contact 44.

En fonctionnement, la grille isolée inférieure est polarisée à une tension Vp proche de la tension de seuil du transistor MOS 24-30-26. Lorsqu'on applique une tension entre les électrodes de source 40 et de drain 42, un faible courant source-drain s'établit du fait de la polarisation de la grille inférieure à une tension proche de la tension de seuil du transistor.

Lorsque le détecteur 20 est placé dans le milieu 39, des molécules 37 du milieu sont susceptibles de s'apparier avec des liaisons pendantes 35 de la grille isolée supérieure 32. A titre d'exemple, on a représenté en figure 2 deux couples 52 constitués chacun d'une molécule 37 associée à une liaison pendante 35. L'étape d'appariement entraîne la génération d'une charge électrique 54 dans la grille 32, à l'interface entre la grille et l'isolant de grille 34. La tension de grille résultante entraîne que le transistor est rendu plus ou moins conducteur, en fonction de la polarisation de la charge induite. En mesurant la variation du courant source-drain, on peut en déduire la concentration des molécules 37 dans le milieu 39.

Selon une caractéristique de l'invention, l'épaisseur de l'isolant de grille 34 est choisie aussi faible que possible. A titre d'exemple, l'épaisseur de l'isolant 34 est comprise entre 0,5 et 5 nm, de préférence entre 2 et 3 nm. Le détecteur 20 est donc très sensible à l'apparition des charges 54. La variation du courant source-drain est ainsi aisément mesurable, et la mesure de concentration obtenue fiable.

Selon une autre caractéristique de l'invention, le rapport entre les épaisseurs respectives de la couche isolante 23 et de l'isolant de grille 34 est choisi aussi élevé que possible. A titre d'exemple, le rapport est compris entre 2 et 20, de préférence entre 8 et 10. La tension de grille du transistor sera plus sensible à une variation de la tension induite par l'appariement des molécules 37 avec les liaisons 35 qu'à une variation de la tension de polarisation Vp. Une faible variation de la tension Vp a peu d'influence sur le courant source-drain. Le bruit sur la mesure de courant dû à la tension Vp est atténué. A titre d'exemple, l'épaisseur de la couche isolante 23 est comprise entre 5 et 50 nm, de préférence entre 15 et 25 nm.

Selon une autre caractéristique de l'invention, l'épaisseur de la région de canal 30 est faible. Ainsi, le couplage capacitif entre la grille supérieure et la grille inférieure est fort. Une variation du nombre de charges sur la grille supérieure impacte donc bien la formation du canal entre la source et le drain sous l'effet de la polarisation de la grille inférieure. A titre d'exemple, l'épaisseur de la région de canal 30 est de préférence comprise entre 1 et 40 nm, de préférence entre 2 et 20 nm.

Pour obtenir une telle épaisseur de région de canal, on utilise avantageusement un substrat de type SOI. La région de canal en silicium monocristallin permet d'obtenir une meilleure conductivité du transistor et donc une meilleure sensibilité du capteur.

De la même manière, en utilisant un substrat de type SOI, les épaisseurs des isolants de grille inférieure et supérieure sont faibles, c'est-à-dire avantageusement inférieures à 60 nm. La sensibilité du détecteur est améliorée.

En choisissant une couche isolante 23 plus épaisse que la couche isolante 34, une variation du nombre de charges dans la région de canal 30 sera détectée et amplifiée par une variation de la tension de grille sur la grille 32 qui sera inférieure à la variation de la tension de grille sur la grille 25, grâce au couplage capacitif entre les grilles supérieure 32 et inférieure 25.

La figure 3 représente un détecteur 60 correspondant à une réalisation pratique du détecteur 20. Le transistor MOS 24-30-26 est entouré d'une tranchée d'isolation 38, par exemple en oxyde de silicium. La tranchée 38 traverse le film mince 21 de silicium, la couche isolante 23 et pénètre dans la tranche 25. La tranchée 38 est suffisamment profonde pour isoler le détecteur 60 d'un détecteur adjacent. A titre d'exemple, la profondeur de la tranchée peut être comprise entre 200 et 400 nm, de préférence entre 250 et 350 nm.

La grille isolée inférieure est constituée d'une partie de la tranche 25 disposée dans un caisson 49. Pour assurer une polarisation efficace de la grille isolée inférieure, le caisson est avantageusement fortement dopé. L'électrode 44 est connectée au caisson 49 par un puits 46 disposé entre la tranchée d'isolation 38 et une tranchée d'isolation adjacente 41. Les électrodes 40, 42 et 44 sont d'une part isolées entre elles et d'autre part isolées du milieu 39 par la présence d'une couche d'isolant 50. A titre d'exemple, ces électrodes peuvent être en tungstène ou en aluminium.

A titre de variante, si la tranche 25 est fortement dopée, on peut prévoir de réaliser la polarisation de la grille inférieure par la face arrière.

Un exemple d'un procédé de fabrication selon un mode de réalisation de la présente invention peut être le suivant.

Sur un substrat de type SOI, on procède à la formation de tranchées d'isolation 38 et 41 traversant la couche de silicium 21 et la couche isolante 23 et pénétrant dans la tranche 25.

On réalise par implantation ionique profonde le caisson 49.

Pour former le puits 46 délimité par les deux tranchées 38 et 41, on grave successivement le film de silicium 21 et la couche isolante 23 sous-jacente. On remplit de silicium le puits 46, par exemple par épitaxie. Cette dernière étape est optionnelle, le contact pouvant se faire directement au fond du puits gravé.

On dépose ensuite l'isolant de grille supérieure 34, qui peut être par exemple de l'oxyde de silicium ou de l'oxyde d'hafnium.

On dépose la grille isolée supérieure 32 puis on réalise les implantations des régions de source 24 et de drain 26, de manière à former par exemple un transistor MOS à canal N.

Après siliciuration des deux régions de drain et de source et du puits 46, on forme les électrodes 40, 42 et 44 dans la couche d'isolant 50.

Le transistor MOS représenté est à canal de type N dans un caisson de type P. En variante, on peut prévoir un transistor à canal P.

Une variante du procédé consiste à réaliser une grille sacrificielle et à former le détecteur jusqu'à la formation des électrodes 40, 42 et 44. La grille sacrificielle est alors retirée et la grille 32 définitive est ensuite déposée dans la cavité. Le matériau formant la grille 32 est fonctionnalisé afin de réagir avec l'environnement. Ainsi, les molécules constituant le matériau ne sont pas dégradées par les diverses étapes propres notamment à la réalisation des régions de source et de drain du transistor.

Une pluralité de détecteurs 60 peuvent être montés en matrice. Dans une matrice donnée, on peut prévoir plusieurs ensembles de détecteurs, chacun des ensembles comprenant uniquement des détecteurs munis d'une couche de détection donnée. On peut ainsi détecter plusieurs types de molécules présentes dans un milieu.

Des modes de réalisation particuliers de la présente invention ont été décrits. Diverses variantes et modifications apparaîtront à l'homme de l'art. En particulier, on a décrit une réalisation dans laquelle le substrat était de type silicium sur isolant. Tout autre type de semi-conducteur sur isolant peut convenir. En outre, le substrat peut être par exemple un substrat massif en matériau semi-conducteur dans lequel on réalise la couche d'isolant de grille 23 par un procédé de type SON (Silicon On Nothing).

De manière non limitative, le détecteur 20 peut servir à la détection de molécules d'ADN ou d'ions présents dans un milieu donné.

Divers modes de réalisation avec diverses variantes ont été décrits ci-dessus. On notera que l'homme de l'art pourra combiner divers éléments de ces divers modes de réalisation et variantes sans faire preuve d'activité inventive.

## Revendications

1. Détecteur (20) de matière biologique ou chimique, comprenant un transistor MOS dont la région de canal en silicium (30) est insérée entre des grilles isolées supérieure (32) et inférieure (25), la grille supérieure étant isolée de la région de canal par un isolant de grille supérieure (34) et la grille inférieure étant isolée du canal par un isolant de grille inférieure (23), la grille isolée supérieure comprenant une couche de détection adaptée à générer une charge à l'interface de la grille isolée supérieure et de son isolant de grille, l'épaisseur de l'isolant de grille supérieure (34) étant inférieure à l'épaisseur de l'isolant de grille inférieure (23).

2. Détecteur selon la revendication 1, dans lequel le rapport entre l'épaisseur de l'isolant de grille inférieure et l'épaisseur de l'isolant de grille supérieure est compris entre 2 et 20, de préférence entre 8 et 10.

3. Détecteur selon la revendication 1 ou 2, dans lequel l'épaisseur de l'isolant de grille supérieure est comprise entre 0,5 et 5 nm, de préférence entre 2 et 3 nm.

4. Détecteur selon l'une quelconque des revendications 1 à 3, dans lequel l'épaisseur de l'isolant de grille inférieure est comprise entre 5 et 50 nm, de préférence entre 15 et 25 nm.

5. Détecteur selon l'une quelconque des revendications 1 à 4, dans lequel l'épaisseur de la région de canal est comprise entre 1 et 40 nm, de préférence entre 2 et 20 nm.

6. Détecteur selon l'une quelconque des revendications 1 à 5, dans lequel la grille isolée inférieure est reliée électriquement à une électrode de contact (44) disposée sur la face avant.

7. Détecteur selon la revendication 6, dont la grille inférieure est constituée d'un caisson (49) fortement dopé relié à l'électrode de contact par un puits (46).

8. Matrice de détecteurs comprenant une pluralité de détecteurs selon l'une quelconque des revendications 1 à 7.

9. Matrice selon la revendication 8, dans laquelle au moins deux détecteurs comprennent une couche de détection distincte.
